(19) Europäisches Patentamt
European Patent Office
Office européen des brevets

(11) EP 1 124 819 B1

(12) **EUROPÄISCHE PATENTSCHRIFT**

(45) Veröffentlichungstag und Bekanntmachung des Hinweises auf die Patenterteilung:
**08.01.2003 Patentblatt 2003/02**

(21) Anmeldenummer: **99953905.9**

(22) Anmeldetag: **21.10.1999**

(51) Int Cl.[7]: **C07D 333/08**, C07D 307/28, C07D 277/22, C07D 209/08, C07D 207/32, C07B 61/00

(86) Internationale Anmeldenummer:
**PCT/EP99/07985**

(87) Internationale Veröffentlichungsnummer:
**WO 00/024732 (04.05.2000 Gazette 2000/18)**

(54) **VERFAHREN ZUR LITHIIERUNG VON FÜNFRINGHETEROCYCLEN**
METHOD OF LITHIATING FIVE-MEMBERED HETEROCYCLES
PROCEDE DE LITHIFICATION D'HETEROCYCLES A CINQ CHAINONS

(84) Benannte Vertragsstaaten:
**BE CH DE FR GB LI**

(30) Priorität: **26.10.1998 DE 19849197**

(43) Veröffentlichungstag der Anmeldung:
**22.08.2001 Patentblatt 2001/34**

(73) Patentinhaber: **Chemetall GmbH**
**60487 Frankfurt (DE)**

(72) Erfinder:
- **LISCHKA, Uwe**
**D-60437 Frankfurt am Main (DE)**
- **HAUK, Dieter**
**D-61169 Friedberg (DE)**
- **RITTMEYER, Peter**
**D-65843 Sulzbach (DE)**
- **WIETELMANN, Ulrich**
**D-61381 Friedrichsdorf (DE)**

(74) Vertreter: **Uppena, Franz, Dr. et al**
**Dynamit Nobel Aktiengesellschaft,**
**Patente, Marken & Lizenzen**
**53839 Troisdorf (DE)**

(56) Entgegenhaltungen:
**WO-A-98/57974**
**GB-A- 1 227 425**
**GB-A- 2 067 997**

- **C.G. SCRETTAS: "On the Mechanism of Ring Metallation of Aromatic Compounds. Metallation of Thiophen by Lithium and by Lithium Dihydroarylides" JOURNAL OF THE CHEMICAL SOCIETY, PERKIN TRANSACTIONS 2,1974, Seiten 745-748, XP002102778 LETCHWORTH GB in der Anmeldung erwähnt**
- **J.J. EISCH ET AL.: "Chemistry of Alkali Metal-unsaturated Hydrocarbon Adducts. I. Metalations with Lithium Metal Adducts of Biphenyl Systems." JOURNAL OF ORGANIC CHEMISTRY, Bd. 27, Nr. 11, 12. November 1962 (1962-11-12), Seiten 3745-3752, XP002102779 EASTON US**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 124 819 B1

Printed by Jouve, 75001 PARIS (FR)

## Beschreibung

Die Erfindung betrifft ein Verfahren zur Lithiierung von CH-aciden Fünfringheterocyclen. bei dem ein Fünfringheterocyclus in einem etherhaltigen Lösungsmittel mit metallischem Lithium in Gegenwart eines H-Akzeptors umgesetzt wird. Die Erfindung betrifft ferner eine Verwendung der Verfahrensprodukte.

Kohlenwasserstoffe lassen sich um so leichter metallieren, je höher ihre CH-Acidität, je elektropositiver das Metall, je größer die aktive Metalloberfläche und je polarer das Lösungsmittel ist. Auf diese Art können Alkine, Cyclopentadien (und Derivate) sowie z.B. Triphenylmethan mittels Alkalimetallen deprotoniert werden. Problematisch ist jedoch, dass Nebenreaktionen, wie z.B. Hydrierungen und/oder CC-Spaltungen, zu schlechten Ausbeuten führen. Diese Nebenreaktionen treten besonders in stark polaren (z.B. Hexamethylphosphorsäuretriamid (HMPT), 1,2-Dimethoxyethan (1,2-DME)) oder protischen Lösungsmitteln (z.B. $NH_3$) in den Vordergrund. In wenig polaren Lösungsmitteln (z.B. Benzol, Ether) ist dagegen die Reaktionsgeschwindigkeit für eine breite Ausnutzung des Direktmetallierungsprinzips zu niedrig. So benötigt man beispielsweise zur Metallierung von Triphenylmethan mit Kalium in siedendem 1,2-DME 10 Stunden. Cäsium nimmt eine Sonderstellung ein, da es beispielsweise mit Toluol bei höheren Temperaturen quantitativ zu unlöslichem Benzylcäsium reagiert.

Fünfringheterocyclen besitzen eine wesentlich niedrigere CH-Acidität als Alkine und Cyclopentadienyle und sind deshalb schwieriger zu metallieren. So liefert Furan nach Umsetzung mit Kalium oder K/Na-Legierung und anschließender Derivatisierung mit $CO_2$ nur kleine Mengen Furan-2-Carbonsäure. Das durch Benzoanellierung aktivierte Thionaphthen setzt sich mit Na um und liefert nach Umsetzung mit $CO_2$ und $H_2O$ das Derivatisierungsprodukt in mäßiger Ausbeute:

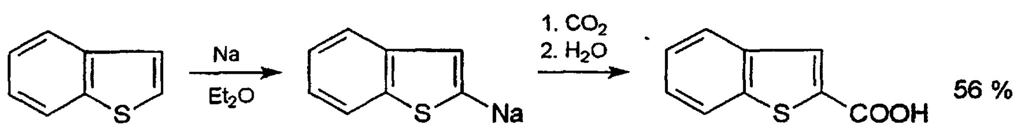


Es ist zu vermuten, dass die schlechten Ausbeuten daraus resultieren, dass Doppelbindungshydrierung auftritt.

Thiophen selbst setzt sich mit Lithiummetalldispersion in THF nur äußerst langsam und mit mäßigen Ausbeuten um. Bei einwöchiger Reaktionszeit wurden von Screttas lediglich 12 % Umsetzung beobachtet (C.G.Screttas, J.C.S. Perkin Transactions II, 1974, 745-748, XP002102778).

An gleicher Stelle wird über Umsetzungen von Lithium mit Thiophen zu Thienyllithium in Gegenwart verschiedener Arene, wie z.B. Naphthalin und/oder $\alpha$-Methylstyrol berichtet. So wurden für die Herstellung von 1 Mol Thienyllithium in Anwesenheit einer etwa stöchiometrischen Menge Naphthalin mindestens 2 Mol Lithium benötigt. Das restliche Lithium, bzw. Lithiumdihydronaphthalenid, wurde durch Nebenreaktionen verbraucht. Im 3. Beispiel der zitierten Literaturstelle (S. 748) wurde die Metallierung in Gegenwart eines großen Thiophen-Überschusses durchgeführt. Die Ausbeute an Thienyllithium betrug bezogen auf eingesetztes Lithium 41 % und bezogen auf eingesetztes Thiophen weniger als 20 %.

Bei der Reaktion von *vorgeformtem* Lithiumdihydronaphthalenid mit überschüssigem Thiophen (4. Beispiel, S. 748) resultierten ebenfalls schlechte Produktausbeuten: 52 % bezogen auf das Lithiumreagenz, bzw. 8 % auf Thiophen. Durch Beimischung bestimmter Kohlenwasserstoffe wie 1,1-Diphenylethylen oder $\alpha$-Methylstyrol konnte die Produktausbeute bei der Umsetzung von Lithiumdihydronaphthalenid mit Thiophen verbessert werden. Im 5. Beispiel (S. 748) stieg die auf das Lithiumreagenz bezogene Ausbeute deutlich auf 95 %. Jedoch wurde Thiophen in großem Überschuß eingesetzt (300 bis 500 %), demzufolge lagen die Metallierungsausbeuten bezogen auf Thiophen unter 50 %. Auch die molare Menge des Hilfsreagenzes Diphenylethylen oder $\alpha$-Methylstyrol überstieg die Lithium-, bzw. Lithiumnaphthalenidmenge um mindestens den Faktor 1,5.

Die Nachteile der von Screttas beschriebenen Synthesen sind generell die äußerst schlechten bis mäßigen Ausbeuten bezogen auf das Lithiumreagenz und/oder vor allem bezogen auf Thiophen. Weiterhin handelt es sich bei den Reaktionen mit Lithiumdihydronaphthalenid um zweistufige Synthesen, bei denen im ersten Schritt nicht stabiles und schwierig handhabbares Lithiumdihydronaphthalenid hergestellt werden muß, das in einem zweiten Schritt mit Thiophen umgesetzt wird. In allen beschriebenen Fällen bilden sich bei den Reaktionen große Mengen an wertlosen Nebenprodukten, nämlich Naphthalin und gegebenenfalls Zersetzungsprodukte.

Die bei der Verwendung von Metallen beobachteten Nebenreaktionen und unerwünschten Nebenprodukte lassen sich vermeiden, wenn als Metallierungsreagenzien Organometallverbindungen wie Butyllithium eingesetzt werden. Butyllithium und andere aus Alkyl- oder Arylhalogeniden hergestellte Lithiumorganyle haben jedoch den Nachteil, dass letztendlich nur max. 50 % des für ihre Synthese eingesetzten Metalls für die 5-Ring-Metallierung verwendet

werden können, da bei ihrer Synthese gemäß

$$R\text{-}Hal + 2\,Li \rightarrow R\text{-}Li + LiHal\downarrow$$

R = Alkyl, Aryl; Hal = Cl, Br, I

50 % des teuren Metalls in ein minderwertiges Salz (LiHal) überführt werden. Sie sind demzufolge teuer.

Von besonderem Interesse sind Organolithiumsynthesen, die das Lithium möglichst quantitativ ausnutzen und in einer einstufigen Reaktion auch möglichst gute Ausbeuten bezogen auf das organische Substrat, in diesem Falle Fünfringheterocyclen, zulassen. Metallierte Fünfringheterocyclen werden in der organischen Synthese sehr häufig verwendet, da sie für die Synthese wertvoller Pharma- und Pflanzenschutzprodukte unverzichtbar sind.

Aufgabe der Erfindung ist es, die Nachteile des Standes der Technik zu beseitigen und ein Verfahren zu schaffen, das ausgehend von metallischem Lithium die direkte, d.h. einstufige, Lithiierung von CH-aciden Fünfringheterocyclen mit hohen Ausbeuten (z.B. 70 % und mehr) erlaubt und es ermöglicht, das eingesetzte Metall möglichst quantitativ für die Deprotonierung auszunutzen, ohne dass wertlose Nebenprodukte, wie z.B. Alkalihalogenide, entstehen. Ferner sollte der Prozeß selektiv verlaufen, d.h. dass nur bestimmte CH-Funktionen des Heterocyclus metalliert werden und keine Hydrierung der im Heterocyclus vorhandenen C=C Doppelbindungen auftritt.

Die Aufgabe wird durch das in den Ansprüchen 1 und 2 angegebene Verfahren gelöst. Die Ansprüche 3 bis 13 bilden das angegebene Verfahren weiter. Die Ansprüche 14 und 15 geben eine besonders vorteilhafte Verwendung der nach dem erfindungsgemäßen Verfahren hergestellten Verbindungen an.

Zur Lithiierung von CH-aciden Fünfringheterocyclen mit einem $pk_a$-Wert von 30 bis 40 wird der Fünfringheterocyclus in einem etherhaltigen Lösungsmittel mit metallischem Lithium in Gegenwart eines Wasserstoffakzeptors (H-Akzeptors) umgesetzt.

Das erfindungsgemäße Verfahren geht von dem in DE 19725192 angegebenen Verfahren aus, bei dem die Direktmetallierung von CH-aciden Verbindungen, die eine oder mehrere CH-Strukturelemente mit $pK_a$-Werten zwischen 10 bis 30 aufweisen, beschrieben wird. Es wurde nun überraschenderweise gefunden, dass auch wesentlich weniger saure elektronenreiche Fünfringheterocyclen mit einem $pK_a$-Wert > 30 bei geeigneter Wahl der Reaktanden und Reaktionsbedingungen mit guter Ausbeute metalliert werden können. Weiter wurde gefunden, dass die Produktausbeuten beim Einsatz der erfindungsgemäßen Wasserstoffakzeptoren mit > 50 % bis nahe 100 % deutlich höher liegen als bei Verwendung von Naphthalin, bzw. Lithiumnaphthalenid mit 41 %.

Als CH-acide Fünfringheterocyclen dienen Verbindungen, die als Ringglieder neben mindestens einer aciden CH-Gruppierung maximal 4 Heteroelemente, ausgewählt aus der Gruppe O, S, N und Se, enthalten. Das sind Fünfringheterocyclen mit einem Heteroatom, wie z.B.

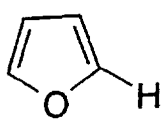
Furan

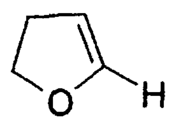
2,3-Dihydrofuran

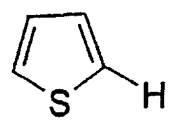
Thiophen

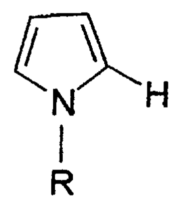
Pyrrol

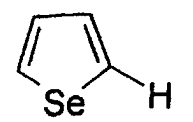
Selenophen

Fünfringheterocyclen mit zwei Heteroatomen, wie z.B.

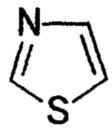

Thiazol

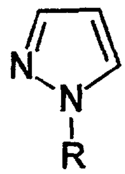

Pyrazol

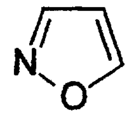

Isoxazol

Fünfringheterocyclen mit drei Heteroatomen, wie z.B.

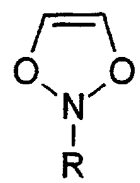

1,3,2-Dioxazol

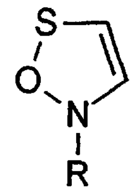

1,2,5-Oxathiazol

oder Fünfringheterocyclen mit vier Heteroatomen, wie z.B.

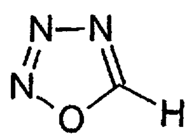

1,2,3,4-Oxatriazol

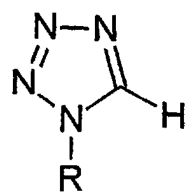

Tetrazol

mit R = H, Alkyl, Aryl,
wobei all diese Verbindungen bis auf diejenigen Spezies, die außer dem CH-aciden Wasserstoffatom kein weiteres Wasserstoffatom am Fünfring aufweisen, auch teilweise substituiert sein können.

Besonders gut eignen sich als CH-acide Fünfringheterocyclen solche 5-gliedrigen Ringsysteme, die mindestens eine olefinische CH-Gruppierung in $\alpha$-Stellung zu einem Heteroatom, ausgewählt aus O, S, N, Se, aufweisen. Das C-Atom der CH-aciden Gruppe ist dabei $sp^2$-hybridisiert.

Die CH-Aciditäten der Fünfringheterocyclen weisen bevorzugt einen $pK_a$-Wert von etwa 30 bis 40 auf. Die nachfolgende Tabelle führt einige Daten auf:

Tabelle 1: CH-Aciditäten

| Verbindung | | $pK_A$-Werte in Cylohexylamin |
|---|---|---|
| Benzol (z. Vgl.) | | 43 |
| 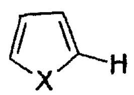 | X = S<br>X = O<br>X = N-Me | 38,4<br>38,1<br>ca.: 38 - 40 |
| 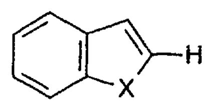 | X = S<br>X = O | 37,1<br>36,8 |
| 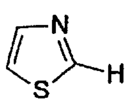 | | 29,5 |
| 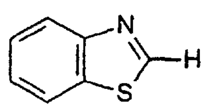 | | 28,1 |

Bei der erfindungsgemäßen Umsetzung wird ein Wasserstoffatom des aciden Fünfringheterocyclus gegen ein Lithiumatom ausgetauscht. Der freiwerdende Wasserstoff wird von einem geeigneten H-Akzeptor aufgenommen. Dabei bilden sich das einfach hydrierte Monomere und/oder das Hydrodimerisierungsprodukt sowie in untergeordnetem Maße höhere Oligomere des Hydrierungsproduktes. Bei der Verwendung von Isopren bildet sich nach gaschromatographischen Befunden beispielsweise Isopenten sowie ein Gemisch verschiedener Dimethyloktadiene mit einem kleinen Anteil höherer Oligomere.

Als geeignete H-Akzeptoren dienen acyclische oder cyclische Diene, wobei 1,3-Diene, wie z.B. Butadien, Isopren oder Cyclohexadien-1,3 bevorzugt sind. Es wurde gefunden, dass 1-Arylolefine, wie Styrol, Methylstyrol oder 1,1-Diphenylethen, nicht in allen Fällen zufriedenstellende Ergebnisse liefern. Die Verwendung von 1-Arylolefinen als H-Akzeptor ist auf die Lithiierung der relativ sauren Fünfringheterocyclen (wie z.B. Thiazol oder andere mehrfach heterosubstituierte Fünfringe) beschränkt. Werden 1-Arylolefine zur Metallierung von weniger aciden Verbindungen wie z.B. Thiophen oder Indol eingesetzt, sind die Ausbeuten deutlich schlechter als bei der Verwendung von 1 3-Dienen.

Der H-Akzeptor wird in einer Menge von 0,2 bis 3 Mol, bevorzugt 0,4 bis 1,5 Mol pro Mol Fünfringheterocyclus eingesetzt. In den meisten Fällen hat sich eine H-Akzeptormenge von 0,5 bis 1,2 Mol pro Mol Fünfringheterocyclus als vorteilhaft erwiesen.

Das für die Metallierung verwendete Lithiummetall sollte bevorzugt in feinverteilter Form, d.h. als Pulver mit Partikelgrößen < 0,1 mm vorliegen. Es lassen sich jedoch auch gröbere Abformungen, z.B. granuliertes Material mit Kantenlängen von wenigen mm einsetzen. Die Reaktionszeiten sind dann aber länger und die Reaktionsausbeuten in der Regel schlechter, es sei denn, dass das grobstückige Lithium im Überschuß eingesetzt wird. Typischerweise wird das Lithium in einer Menge von 0,5 bis 3 Mol, bevorzugt 1 bis 1,5 Mol, pro Mol Fünfringhetreocyclus eingesetzt. Beim Einsatz von feinverteiltem Lithium genügt eine weitgehend stöchiometrische Menge von 0,95 bis 1,1 Mol Li pro

Mol Fünfringheterocyclus.

Als Lösungsmittel werden offenkettige oder zyklische Monoether, insbesondere Tetrahydrofuran (THF) oder Methyl-tert.-Butylether (MTBE), oder Polyether, wie z.B. 1,2-Dimethoxyethan (1,2-DME) oder Diethylenglykoldimethylether, eingesetzt. Diese können in reiner Form oder im Gemisch untereinander oder im Gemisch mit Kohlenwasserstoffen, wie z.B. Pentan, Hexan, Cyclohexan, Methylcyclohexan, Heptan, Octan, Toluol oder Ethylbenzol, vorliegen. Da Kohlenwasserstoffe im allgemeinen deutlich preisgünstiger sind als etherische Lösungsmittel, bedeutet ein Kohlenwasserstoffanteil im Lösungsmittel eine Steigerung der Wirtschaftlichkeit des erfindungsgemäßen Verfahrens.

Es wurde beobachtet, dass in einigen Fällen, insbesondere bei den wenig CH-aciden Fünfringheterocyclen, wie z.B. 2,3-Dihydrofuran, die Reaktion nur sehr verzögert einsetzt und/oder nur mit mäßigen Ausbeuten abläuft. Speziell in diesen Fällen empfiehlt es sich, das Metall in bekannter Art und Weise zu aktivieren. Besonders gut ist dafür die Zugabe eines Metallphasentransferkatalysators, im folgenden Phasentransferkatalysator (PTC) genannt, wie z.B. Naphthalin, Anthracen, Diphenyl oder Di-tert-butyldiphenyl, geeignet. Die genannten polycyclischen Aromaten vermögen in wasserfreien polar-aprotischen Lösungsmitteln Lithium unter Bildung von Radikalanionenkomplexen zu addieren. Dadurch wird die Oxidschicht auf dem Metall aufgebrochen und Lithium in eine hochreaktive lösliche Form überführt. Die genannten Katalysatoren vermindern auf diese Weise die unerwünschte Induktionsphase; außerdem führt ihre Anwesenheit im Reaktionsgemisch dazu, dass zum Erlangen einer bestimmten Produktausbeute weniger H-Akzeptor benötigt wird. Die Zugabemenge des PTC beträgt typischerweise 0 bis 0,2 Mol, bevorzugt 0 bis 0,1 Mol, pro Mol Fünfringheterocyclus.

Im allgemeinen ist das experimentelle Vorgehen wie folgt:

Zunächst wird das Lithiummetall in dem wasserfreien, aprotischen Lösungsmittel oder Lösungsmittelgemisch suspendiert. Die Metallsuspension wird dann mit dem zu metallierenden Fünfringheterocyclus versetzt. Im Prinzip ist auch das umgekehrte Vorgehen (d.h. Zudosierung der Li-Suspension oder des Lithiums zur Lösung des Fünfringheterocyclus) möglich. Diese Variante erweist sich jedoch als technisch aufwendiger.

Die Metallierungsreaktion wird anschließend durch Zugabe des H-Akzeptors gestartet. Bei Einsatz eines Phasentransferkatalysators kann dieser in unterschiedlicher Weise zugegeben werden. Besonders vorteilhaft wird er mit dem Lithium zugegeben. Er kann aber auch in Mischung mit dem H-Akzeptor eingebracht werden.

Die günstigsten Reaktionstemperaturen liegen in der Regel zwischen 0 und 60 °C, wobei thermisch labile Produktlösungen bei tieferen.Temperaturen hergestellt werden. Höhere Temperaturen führen tendenziell zur Produktzersetzung; tiefere sind tendenziell wegen ihres höheren Energieverbrauchs weniger wirtschaftlich.

Die Eindosierungszeiten liegen in Abhängigkeit vom Fünfringheterocyclus und der Kühlkapazität zwischen etwa 15 Minuten und wenigen Stunden. Nach Beendigung der H-Akzeptorzugabe schließt sich eine Nachreaktionsphase an, die im allgemeinen 15 Minuten bis 4 Stunden in Anspruch nimmt. Nach Beendigung der Nachreaktion wird die Reaktionsmischung filtriert, um Reste nicht abreagierten Metalls und geringe Mengen unlöslicher Nebenprodukte zu entfernen.

Die mit diesem Verfahren erzielbaren Ausbeuten an selektiv lithiiertem Fünfringheterocyclus sind abhängig von der CH-Acidität sowie von reaktionsspezifischen Parametern (z.B. Art des Lösungsmittels, Stöchiometrie, Katalysatoreinsatz) und liegen zwischen 30 und nahezu 100 %.

Erhalten wird eine Produktlösung, die ca. 5 bis 25 Gew.% des lithiierten Fünfringheterocyclus, hydrierte Dimere und Oligomere des H-Akzeptors und gegebenenfalls Reste des PTC enthält.

Es wurde beobachtet, dass einige erfindungsgemäß hergestellte Produktlösungen, z.B. 2-Furanyllithium, in reinen etherischen Lösungsmitteln nicht ausreichend lagerstabil sind. Während sich beispielsweise eine ca. 11 Gew. %ige 2-Furanyllithiumlösung mit einem Molverhältnis 2-Furanyl-Li : THF von ca. 1 : 7 bei 25 °C mit einer Rate von etwa 10 % pro Tag zersetzt, liegt die Zersetzungsrate für eine ebenfalls 11 Gew.%ige 2-Furanyllithiumlösung, in der ein Teil des THF durch einen Kohlenwasserstoff (z.B. Toluol oder Cyclohexan) ersetzt wurde und die ein Molverhältnis 2-Furanyl-Li : THF von ca. 1 : 1 aufweist, bei nur 0,12 % pro Tag. Eine THF-arme 2-Furanyllithiumlösung ist demnach wesentlich stabiler und läßt sich länger bzw. ohne aufwendige Kühlmaßnahmen lagern und transportieren.

Die fertigen Produktlösungen, wie z.B. 2-Lithio-Furan, im folgenden Furanyllithium genannt, oder 2-Lithio-Thiophen, im folgenden Thienyllithium genannt, können durch Umsetzung mit Elektrophilen, wie z.B. Carbonylverbindungen, Oxiranen, Schwefel, Kohlendioxid oder Alkylhalogeniden, derivatisiert werden. Diese Produkte finden vielfältigen Einsatz in der organischen Chemie, insbesondere als Zwischenprodukte zur Herstellung von Pharma- und Pflanzenschutzpräparaten.

Der Gegenstand der Erfindung wird nachfolgend anhand von Ausführungsbeispielen näher erläutert.

**Beispiele 1 bis 10**

Die Beispiele 1 bis 10 (dargestellt in Tabelle 2) zeigen die Herstellung von 2-Thienyllithium aus Thiophen mittels unterschiedlicher Verfahrensvarianten. Thiophen ist mit einem $pK_a$-Wert von 38,4 nur wenig saurer als Toluol (40,9). In den Beispielen 1 bis 5 und 7 bis 10 wurde nach folgender allgemeiner Arbeitsvorschrift vorgegangen:

Das Lithiumpulver (Korngröße < 0,1 mm) wurde im angegebenen Lösungsmittel suspendiert und gegebenenfalls mit einem Phasentransferkatalysator (PTC) versetzt. Nach Zugabe von 68 g (1,0 Mol) Thiophen wurde innerhalb von 1 bis 2 Std. der jeweilige H-Akzeptor zugetropft (Styrol, Isopren) bzw. eingeleitet (Butadien). Nach einer 0,5 bis 4 stündigen Nachreaktionszeit wurden die Reaktionsgemische klarfiltriert und die Ausbeute mittels Basentitration bzw. mittels quantitativer Gaschromatographie (GC) bestimmt. Dabei wurde im GC 2-Trimethylsilylthiophen (nach Derivatisierung des Produktes mit Trim- ethylchlorsilan) gemessen.

In Beispiel 6 wurde analog vorgegangen. Allerdings wurde der Phasentransferkatalysator nicht im Lösungsmittel vorgelegt, sondern zusammen mit dem H-Akzeptor zudosiert.

Tabelle 2:

| Herstellung von 2-Thienyllithium | | | | | | | | |
|---|---|---|---|---|---|---|---|---|
| Bsp. | Molverhältnis [1] | | PTC[1)3)] | Mol Lösungsmittel pro Mol Thiophen | | HerstellTemp. [4] [°C] | Ausbeute (%) | |
| | Li | H-Akzepto[2] | [Mol%] | | | | Gesamtbase[5] | GC[6] |
| 1 | 0,95 | 0,49 S | / | 3,0 | THF | ca. 25 | 5 | 3 |
| 2 | 0,99 | 0,49 S | 6,9 N | 3,0 | THF | ca. 25 | 73 | 47 |
| 3 | 1,1 | 0,58 I | / | 6,6 | THF | 25/50 | 56 | 61 |
| 4 | 0,97 | 0,61 I | 1,0 N | 6,9 | THF | ca. 23 | 70 | 73 |
| 5 | 0,97 | 0,62 I | 3,1 N | 6,9 | THF | ca. 23 | 93 | 82 |
| 6 | 1,1 | 0,60 I | 3,1 A | 6,7 | THF | 25/50 | 65 | 61 |
| 7 | 0,97 | 0,63 I | 2,9 N | 2,0<br>4,1 | THF/<br>Cyclohexan | ca. 23 | 84 | 80 |
| 8 | 0,97 | 0,75 I | 2,0 N | 2,0<br>3,1 | THF/<br>Toluol | 35 | 93 | / |
| 9 | 0,96 | 0,59 I | 3,0 N | 5,3 | 1,2-DME | ca. 23 | 78 | / |
| | 0,94 | 1,0 B | / | 2,0<br>2,8 | THF/<br>Toluol | ca. 10 | 87 | 90 |

[1] Thiophen = 1;
[2] S = Styrol; I = Isopren; B = 1,3-Butadien;
[3] N = Naphthalin; A = Anthracen;
[4] Vor-/Nachreaktion
[5] Gesamtbasenmenge in Lösung;
[6] Gaschromatographische Bestimmung als 2-Trimethylsilylthiophen

Tabelle 2 zeigt folgende Ergebnisse:

Wird als H-Akzeptor Styrol eingesetzt und ohne Phasentransferkatalysator (PTC) gearbeitet, so wird 2-Thienyllithium nur in sehr geringer Ausbeute (< 5%, Beispiel 1) erhalten. Unter gleichen Bedingungen führt die Zugabe von 6,9 Mol% Naphthalin als PTC zu einer deutlichen Ausbeutesteigerung (Beispiel 2). Wird anstelle von Styrol Isopren eingesetzt, ist auch ohne PTC eine Produktausbeute von > 50 % zu erreichen (Beispiel 3).

Bei gleichzeitiger Zugabe von Naphthalin als PTC wird eine weitere Ausbeutesteigerung registriert (Beispiele 4 und 5). Anstelle von Naphthalin können auch andere polycyclische Aromaten, beispielsweise Anthracen (Beispiel 6) als PTC eingesetzt werden.

Das relativ teure THF kann teilweise durch billigere Kohlenwasserstoffe wie Cyclohexan oder Toluol ersetzt werden, ohne dass ein negativer Einfluß auf die Ausbeute zu beobachten ist (Beispiele 7 und 8). Allerdings sollten in der Produktlösung mindestens 2 Mol eines etherischen Lösungsmittels, vorzugsweise THF, pro Mol 2-Thienyllithium anwesend sein, um kristallisationsstabile Lösungen zu erhalten.

Statt ihn vorzulegen kann der PTC auch zusammen mit dem H-Akzeptor zugegeben werden (Beispiel 8). Beispiel 9 beschreibt die Verwendung von 1,2-Dimethoxyethan als Reaktionslösungsmittel. Beispiel 10 zeigt, dass bei Verwendung einer größeren Menge H-Akzeptor (in diesem Falle 1,3-Butadien) auch ohne PTC-Einsatz sehr gute Produktausbeuten erreicht werden können.

**Beispiele 11 bis 16**

Die Beispiele 11 bis 16 (dargestellt in Tabelle 3) zeigen die Lithiierung diverser Fünfringheterocyclen. Dabei wurde nach folgender allgemeiner Arbeitsvorschrift vorgegangen:

Das Lithiumpulver (Korngröße < 0,1 mm) wurde im angegebenen Lösungsmittel suspendiert, mit dem Phasentransferkatalysator Naphthalin versetzt und nach Beginn der Grünfärbung mit 0,5 Mol des jeweiligen CH-aciden Fünfringheterocyclus versetzt. Dann wurde innerhalb von 1 bis 2 Std. der H-Akzeptor Isopren zugetropft. Nach einer ca. 1 stündigen Nachreaktionszeit wurden das Reaktionsgemisch klar filtriert, das Produkt nach Derivatisierung mit Methyliodid gaschromatographisch (GC) und massenspektroskopisch (MS) charakterisiert und die Ausbeute mittels Basentitration bestimmt.

Tabelle 3: Lithiierung diverser Fünfringheterocyclen

| Bsp. | Heterocyclus | Molverhältnis [1] Li | Isopren | PTC [1)2)] (Mol%) | Lösungsmittel Mol / Mol Heterocycl. | Derivatisierungsprodukt [3] | Ausbeute (%) [4] |
|---|---|---|---|---|---|---|---|
| 11 | 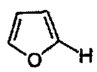 | 0,91 | 0,66 | 3 | 0,9 THF/ 5,3 Toluol | 2-Me-furan | 97 |
| 12 | 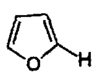 | 0,90 | 0,68 | 1 | 0,9 THF/ 5,9 Toluol | 2-Me-furan | 65 |
| 13 | 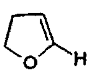 | 1,00 | 0,99 | 3 | 6,1 THF | 2-Me-Dihydrofuran | 60 |
| 14 | 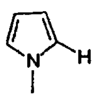 | 0,95 | 0,98 | 2,9 | 5,5 1,2-DME | 1,2-Dimethylpyrrol | 82 |
| 15 | 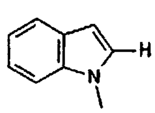 | 1,00 | 1,03 | 3,2 | 12,0 THF | 1,2-Dimethylindol | 69 |
| 16 | 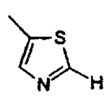 | 1,00 | 0,51 | 2,9 | 8,7 THF | / | 86 |

[1] Heterocyclus = 1; [2] PTC = Naphthalin [3] Umsetzung mit Methyliodid und Identifizierung durch GC/MS; [4] Bestimmung der in Lösung befindlichen Alkalität nach Filtration

Tabelle 3 zeigt folgende Ergebnisse:

Mit sehr guten Ausbeuten läßt sich Furan lithiieren, wobei Naphthalin die Produktbildung begünstigt (Beispiele 11 und 12). Das weniger acide 2,3-Dihydrofuran ergibt geringere Ausbeuten, obwohl mehr H-Akzeptor eingesetzt wurde (Beispiel 13). N-Methylpyrrol wird in 1,2-Dimethoxyethan in guten Ausbeuten zu 2-Lithio-N-Methylpyrrol umgesetzt (Beispiel 14). N-Methylindol läßt sich in THF mit zufriedenstellenden Ausbeuten lithiieren (Beispiel 15). Für die Lithiierung des relativ sauren 4-Methylthiazol genügt bereits eine geringere Menge Wasserstoffakzeptor (Beispiel 16).

## Patentansprüche

1. Verfahren zur Lithiierung von CH-aciden Fünfringheterocyclen, **dadurch gekennzeichnet. dass** der Fünfringheterocyclus in einem etherhaltigen Lösungsmittel mit metallischem Lithium in Gegenwart eines H-Akzeptors umgesetzt wird, wobei die CH-acide Bindung des Fünfringheterocyclus einen $pk_a$-Wert von 30 bis 40 aufweist und wobei der H-Akzeptor
ein offenkettiges, nicht substituiertes oder substituiertes
1,3-Dien

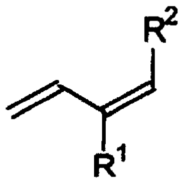

mit $R^1$, $R^2$ = H, Alkyl, Vinyl
($R^1$, $R^2$ in cis- oder trans-Stellung)
oder ein zyklisches 1,3-Dien

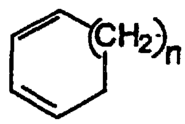

mit n = 1 bis 5
ist und in einer Menge von 0,2 bis 3 Mol pro Mol Fünfringheterocyclus eingesetzt wird.

2. Verfahren zur Lithiierung von CH-aciden mehrfach heterosubstituierten Fünfringen, **dadurch gekennzeichnet, dass** der Fünfringheterocyclus in einem etherhaltigen Lösungsmittel mit metallischem Lithium in Gegenwart eines H-Akzeptors umgesetzt wird, wobei die CH-acide Bindung des Fünfringheterocyclus einen $pk_a$-Wert von 30 bis 40 ausweist und wobei der H-Akzeptor
ein offenkettiges, nicht substituiertes oder substituiertes
1,3-Dien

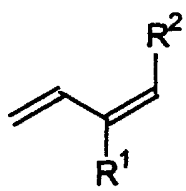

mit $R^1$, $R^2$ = H, Alkyl, Vinyl
($R^1$, $R^2$ in cis- oder trans-Stetlung)
oder ein zyklisches 1,3-Dien

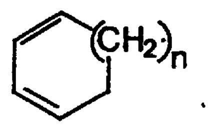

mit n = 1 bis 5
oder ein nicht substituiertes oder substituiertes 1-Arylolefin

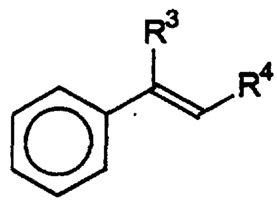

mit $R^3$, $R^4$ = H, Alkyl (mit 1 bis 5 C-Atomen)
($R^3$, $R^4$ in cis- oder trans-Stellung)
ist und in einer Menge von 0,2 bis 3 Mol pro Mol Fünfringheterocyclus eingesetzt wird.

**3.** Verfahren nach Anspruch 1 oder 2, **dadurch gekennzeichnet, dass** der Fünfringheterocyclus in α-Stellung zu mindestens einem Ringheteroatom (O, S, N oder Se) mindestens eine CH-acide Gruppe, deren C-Atom $sp^2$-hybridisiert ist, aulweist.

**4.** Verfahren nach Anspruch 1 oder 3, **dadurch gekennzeichnet, dass** der Fünfringheterocyclus Furan, 2,3-Dihydrofuran, Thiophen oder Pyrrol ist und wobei diese, Verbindung bis auf eine in α-Stellung zum Ringheteroatom befindliche CH-acide Gruppe beliebig substituiert sein kann.

**5.** Verfahren nach einem der Ansprüche 1 bis 4, **dadurch gekennzeichnet, dass** der H-Akzeptor Isopren, Butadien oder cyclohexadien-1,3 ist.

**6.** Verfahren nach einem der Ansprüche 1 bis 5, **dadurch gekennzeichnet, dass** der H-Akzeptor in einer Menge von 0,4 bis 1,5 Mol pro Mol Fünfringheterocyclus eingesetzt wird.

**7.** Verfahren nach einem der Ansprüche 1 bis 6, **dadurch gekennzeichnet, dass** das Lithiummetall in feinverteilter Form, d.h. als Pulver mit Komgrößen < 0,1 mm eingesetzt wird.

**8.** Verfahren nach einem der Ansprüche 1 bis 7, **dadurch gekennzeichnet, dass** als Lösungsmittel ein oder mehrere offenkettige oder cyclische Ether oder Gemische aus einem oder mehreren Ethem und einem oder mehreren Kohlenwasserstoffen eingesetzt werden.

**9.** Verfahren nach Anspruch 8, **dadurch gekennzeichnet, dass** als Lösungsmittel THF in reiner Form oder gemischt mit Kohlenwasserstoffen eingesetzt wird.

**10.** Verfahren nach Anspruch 8 oder 9, **dadurch gekennzeichnet, dass** der Kohlenwasserstoff Pentan, Hexan, Cyclohexan, Methylcyclohexan, Heptan, Octan, Toluol oder Ethylbenzol ist.

**11.** Verfahren nach einem der Ansprüche 1 bis 10, **dadurch gekennzeichnet, dass** die Reaktion in Gegenwart eines Metallphasentransferkatalysators durchgeführt wird, wobei der Metallphasentransferkatalysator in einer Menge bis zu 0,2 Mol pro Mol Fünfringheterocyclus eingesetzt wird.

**12.** Verfahren nach Anspruch 11, **dadurch gekennzeichnet, dass** der Metallphasentransferkatalysator ein polycyclischer Aromat ist.

13. Verfahren nach Anspruch 12, **dadurch gekennzeichnet, dass** der polycyclische Aromat Naphthalin, Anthracen, Diphenyl oder Di-tert-butyldiphenyl ist.

14. Verwendung der nach dem Verfahren der Ansprüche 1 bis 13 hergestellten lithiierten Fünfringheterocyclen zur Umsetzung mit elektrophilen Verbindungen.

15. Verwendung der nach dem Verfahren der Ansprüche 1 bis 13 hergestellten lithiierten Fünfringheterocyclen zur Herstellung von Zwischenprodukten in der Synthese von Pharma- und Pflanzenschutzpräparaten.

## Claims

1. Method of lithiating CH-acidic five-membered heterocycles, **characterised in that** the five-membered heterocycle is reacted with metallic lithium in an ether-containing solvent in the presence of an H acceptor, and the CH-acidic bond of the five-membered heterocycle has a $pK_a$ value of 30 to 40 and the H acceptor is
an open-chain, unsubstituted or substituted 1,3-diene

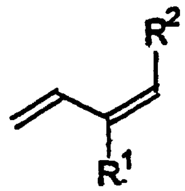

wherein $R^1$, $R^2$ = H, alkyl, vinyl
($R^1$, $R^2$ in cis or trans position)
or a cyclic 1,3-diene

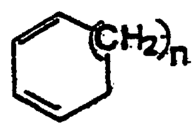

wherein n = 1 to 5
and is used in a quantity of 0.2 mol to 3 mol per mol of five-membered heterocycle.

2. Method of lithiating CH-acidic multiply heterosubstituted five-membered rings, **characterised in that** the five-membered heterocycle is reacted with metallic lithium in an ether-containing solvent in the presence of an H acceptor, and the CH-acidic bond of the five-membered heterocycle has a $pK_a$ value of 30 to 40 and the H acceptor is an open-chain, unsubstituted or substituted 1,3-diene,

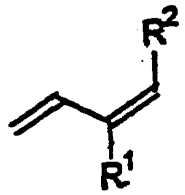

wherein $R^1$, $R^2$ = H, alkyl, vinyl
($R^1$, $R^2$ in cis or trans position)
or a cyclic 1,3-diene

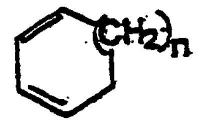

wherein n = 1 to 5
or an unsubstituted or substituted 1-arylolefin

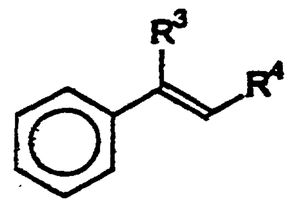

wherein $R^3$, $R^4$ = H, alkyl (with 1 to 5 C atoms) ($R^3$, $R^4$ in cis or trans position)
and is used in a quantity of 0.2 mol to 3 mol per mol of five-membered heterocycle.

**3.** Method according to claim 1 or 2, **characterised in that**, in the α-position to at least one ring hetero atom (O, S, N or Se), the five-membered heterocycle has at least one CH-acidic group in which the C atom is $sp^2$-hybridised.

**4.** Method according to one of claims 1 to 3, **characterised in that** the five-membered heterocycle is furan, 2,3-dihydrofuran, thiophene or pyrrole and that this compound can be substituted as desired., except for a CH-acidic group situated in the α-position to the ring hetero atom.

**5.** Method according to one of claims 1 to 4, **characterised in that** the H acceptor is isoprene, butadiene or 1,3-cyclohexadiene.

**6.** Method according to one of claims 1 to 4, **characterised in that** the H acceptor is used in a quantity of 0.4 to 1.5 mol per mol of the five-membered heterocycle.

**7.** Method according to one of claims 1 to 6, **characterised in that** the lithium metal is used in finely divided form, i. e. as powder having particle sizes of < 0.1 mm.

**8.** Method according to one of claims 1 to 7, **characterised in that** one or more open-chain or cyclic ethers, or mixtures of one or more ethers and of one. or more hydrocarbons, are used as solvent.

**9.** Method according to claim 8, **characterised in that** THF in pure form or mixed with hydrocarbons is used as the solvent.

**10.** Method according to claim 8 or 9 **characterised in that** the hydrocarbon is pentane, hexane, cyclohexane, methylcyclohexane, heptane, octane, toluene or ethylbenzene.

**11.** Method according to one of claims 1 to 10, **characterised in that** the reaction is carried out in the presence of a metal phase transfer catalyst, the metal phase transfer catalyst being used in a quantity of up to 0.2 mol per mol of five-membered heterocycle.

**12.** Method according to claim 11, **characterised in that** the metal phase transfer catalyst is a polycyclic aromatic.

**13.** Method according to claim 12, **characterised in that** the polycyclic aromatic is naphthalene, anthracene, diphenyl or di-tert.-butyldiphenyl.

**14.** Use of the lithiated five-membered heterocycles produced by the method according to claims 1 to 13 for reaction

with electrophilic compounds.

**15.** Use of the lithiated five-membered heterocycles produced by the method according to claims 1 to 13 for the preparation of intermediate products in the synthesis of pharmaceuticals and plant protection products.

## Revendications

**1.** Procédé de lithiation d'hétérocycles à 5 chaînons comportant un groupe CH acide, **caractérisé par le fait que** l'on fait réagir l'hétérocycle à 5 chaînons dans un solvant contenant de l'éther avec du lithium métallique, en présence d'un accepteur de protons, la liaison CH acide de l'hétérocycle à 5 chaînons ayant un $pK_a$ compris entre 30 et 40 et l'accepteur de protons étant un 1,3-diène à chaîne ouverte, substitué ou non-substitué, de formule

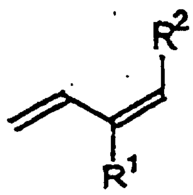

dans laquelle $R^1$, $R^2$ = H, alkyle, vinyle, ($R^1$ et $R^2$ étant en position *cis* ou *trans* l'un par rapport à l'autre),
ou un 1,3-diène cyclique de formule

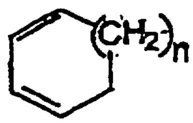

dans laquelle n est compris entre 1 et 5, et l'accepteur de protons étant utilisé en une quantité comprise entre 0,2 et 3 moles par mole d'hétérocycle à 5 chaînons.

**2.** Procédé de lithiation d'hétérocycles à 5 chaînons polysubstitués à groupe CH acide, **caractérisé par le fait que** l'on fait réagir l'hétérocycle à 5 chaînons dans un solvant contenant de l'éther avec du lithium métallique, en présence d'un accepteur de protons, la liaison CH acide de l'hétérocycle à 5 chaînons ayant un $pK_a$ compris entre 30 et 40 et l'accepteur de protons étant un 1,3-diène à chaîne ouverte, substitué ou non-substitué, de formule

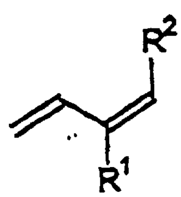

dans laquelle $R^1$, $R^2$ = H, alkyle, vinyle, ($R^1$ et $R^2$ étant en position *cis* ou *trans* l'un par rapport à l'autre),
ou un 1,3-diène cyclique de formule

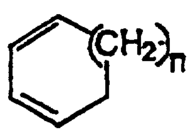

dans laquelle n est compris entre 1 et 5, ou une 1-aryloléfine, substituée ou non-substituée, de formule

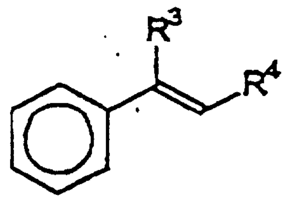

dans laquelle $R^3$, $R^4$ = H, alkyle en $C_{1-5}$, ($R^3$ et $R^4$ étant en position *cis* ou *trans* l'un par rapport à l'autre), l'accepteur de protons étant utilisé en une quantité comprise entre 0,2 et 3 moles par mole d'hétérocycle à 5 chaînons.

3. Procédé selon la revendication 1 ou 2, **caractérisé par le fait que** l'hétérocycle à 5 chaînons présente en position $\alpha$ par rapport à au moins un hétéroatome (O, S, N ou Se) du cycle au moins un groupe CH acide dont l'atome de carbone est hybridé $sp^2$.

4. Procédé selon la revendication 1 ou 3, **caractérisé par le fait que** l'hétérocycle à 5 chaînons est un cycle furane, 2,3-dihydrofurane, thiophène ou pyrrole, tous les atomes du cycle, à l'exception du groupe CH en position $\alpha$ de l'hétéroatome, pouvant être substitués.

5. Procédé selon une des revendications 1 à 4, **caractérisé par le fait que** l'accepteur de protons est l'isoprène, le butadiène ou le cyclohexadiène-1,3.

6. Procédé selon l'une des revendications 1 à 5, **caractérisé par le fait que** l'accepteur de protons est utilisé en une quantité comprise entre 0,4 et 1,5 moles par mole d'hétérocycle à 5 chaînons.

7. Procédé selon l'une des revendications 1 à 6, **caractérisé par le fait que** le lithium métallique est utilisé sous une forme finement divisée, c'est-à-dire sous forme d'une poudre ayant une granulométrie inférieure à 0,1 mm.

8. Procédé selon l'une des revendications 1 à 7, **caractérisé par le fait que** l'on utilise, comme solvant, un ou plusieurs éthers cycliques ou à chaîne ouverte ou un mélange d'un ou de plusieurs éthers et d'un ou de plusieurs hydrocarbures.

9. Procédé selon la revendication 8, **caractérisé par le fait que** l'on utilise, en tant que solvant, du THF pur ou du THF mélangé avec un ou plusieurs hydrocarbures.

10. Procédé selon la revendication 8 ou 9, **caractérisé par le fait que** l'hydrocarbure est choisi parmi le pentane, l'hexane, le cyclohexane, le méthylcyclohexane, l'heptane, l'octane, le toluène ou l'éthylbenzène.

11. Procédé selon une des revendications 1 à 10, **caractérisé par le fait que** l'on met en oeuvre la réaction en présence d'un catalyseur métallique de transfert de phase, le catalyseur métallique de transfert de phase étant utilisé en une quantité allant jusqu'à 0,2 mole par mole d'hétérocycle à cinq chaînons.

12. Procédé selon la revendication 11, **caractérisé par le fait que** le catalyseur métallique de transfert de phase est un composé polycyclique aromatique.

13. Procédé selon la revendication 12, **caractérisé par le fait que** le composé polycyclique aromatique est choisi parmi le naphtalène, l'anthracène, le diphényle ou le di-tert-butyldiphényle.

14. Utilisation des composés hétérocycliques à 5 chaînons lithiés obtenus selon le procédé selon l'une des revendications 1 à 13, pour la réaction avec des composés électrophiles.

15. Utilisation des composés hétérocycliques à 5 chaînons lithiés obtenus selon le procédé selon l'une des revendications 1 à 13, pour la préparation de produits intermédiaires pour la synthèse de préparations pharmaceutiques et phytosanitaires.